# EUROPÄISCHE PATENTSCHRIFT

(11) **EP 3 556 538 B1**
(45) Veröffentlichungstag und Bekanntmachung des Hinweises auf die Patenterteilung: **26.04.2023**
(21) Anmeldenummer: 19163351.0
(22) Anmeldetag: 18.03.2019
(51) Int. Cl.: B29C 55/24, B29C 67/00, B29L 31/00

(54) **VERFAHREN ZUM HERSTELLEN EINER INJEKTIONSVORRICHTUNG MIT BYPASSKANAL UND WERKZEUG HIERFÜR**
METHOD FOR PRODUCING AN INJECTION DEVICE WITH BYPASS CHANNEL AND TOOL USED FOR THIS PURPOSE
PROCÉDÉ DE FABRICATION D'UN DISPOSITIF D'INJECTION POURVU DE CANAL DE DÉRIVATION ET OUTIL ASSOCIÉ

(30) Priorität: 11.04.2018 DE 102018108549
(43) Veröffentlichungstag der Anmeldung: 23.10.2019
(73) Patentinhaber: Gerresheimer Regensburg GmbH, 93047 Regensburg (DE)
(72) Erfinder: KALB, Franz, 92536 Pfreimd (DE); LANZL, Christian, 93170 Bernhardswald (DE)
(74) Vertreter: Reichert & Lindner Partnerschaft Patentanwälte

(56) Entgegenhaltungen:
- DE-A1-102007 014 281
- DE-A1-102011 107 764
- FR-A1- 2 244 609
- US-A- 3 932 093

## Beschreibung

Die Erfindung betrifft ein Verfahren zum Herstellen einer Injektionsvorrichtung mit Bypasskanal.

Ferner betrifft die Erfindung ein Werkzeug für das Verfahren zum Herstellen einer Injektionsvorrichtung mit Bypasskanal.

Die deutsche Patentanmeldung DE 10 2007 014 281 A1 offenbart ein Verfahren zum Herstellen einer Mehrkammer-Spritze mit Bypasskanal. Hierzu wird zunächst ein Vorformling aus einem thermoformbaren Kunststoff mit einem rohrförmigen Abschnitt hergestellt. Der Vorformling wird in dem Bereich, in dem der Bypasskanal ausgebildet werden soll, mit einer Matrize umschlossen. Die Matrize hat in dem Bereich des Bypasskanals eine Vertiefung ausgebildet. Der Vorformling wird erwärmt und zwar auf eine Temperatur oberhalb des Erweichungsbereiches des Kunststoffs. Der rohrförmige Abschnitt des Vorformlings wird in der Matrize positioniert und mit Druck beaufschlagt. Aufgrund der Druckdifferenz wird die Wandung des Vorformlings in die Vertiefungen der Matrize gedrückt, so dass dadurch der Bypasskanal plastisch ausgeformt wird. Der Nachteil dieses Verfahrens ist, dass sich mit dem zusätzlichen Werkzeug (Matrize) und dem erforderlichen Anlegen eines Drucks im Vorformling die Produktionskosten und die Zykluszeit für die Herstellung einer Mehrkammer-Spritze mit Bypasskanal erhöhen. DE 10 2007 014 281 A1 offenbart ebenfalls ein Verfahren und ein Werkzeug zum Herstellen einer Injektionsvorrichtung mit einem Bypasskanal vermittels einer Matrize.

Die deutsche Offenlegungsschrift DE 29 25 858 offenbart einen Faltkern, mit dem Hinterschneidungen im Innern von Spritzgussteilen ausgebildet werden können. Mit dem Faltkern könnte auch der Bypasskanal in der Injektionsspritze geformt werden. Der Faltkern ist jedoch mechanisch aufwändig und würde beim Spritzgussprozess der Injektionssspritze doch auch zu Trennkanten und Partikelbildung führen.

Eine weitere Möglichkeit der Ausbildung des Bypasskanals in der Injektionsspritze ist, dass man Einlegeteile verwendet, die beim Spritzgussprozess den Bypasskanal formen. Hierzu würde ein zweites Werkzeug benötigt, was zu längeren Zyklen bei der Herstellung von Injektionsspritzen führt. Diese längeren Zyklen und das zweite Werkzeug erhöhen folglich die Teilepreise für die Injektionsspritze, die mit dem Bypasskanal versehen ist. Das Handling mit den Einlegeteilen führt ebenfalls zu längeren Zykluszeiten, höheren Kosten und die Partikelbildung kann beim Spritzgussprozess nicht ausgeschlossen werden.

Der Bypasskanal in der Injektionsspritze könnte auch mittels eines Zweikomponenten-Spritzgussverfahrens hergestellt werden. Der Zweikomponenten-Spritzguss hat jedoch den Nachteil, dass dieser zu höheren Werkzeugkosten und zu einer niedrigeren Belegung des Werkzeugs führt. Das Werkzeug selbst ist sehr komplex und somit auch anfällig. Letztendlich führt der Zweikomponenten-Spritzguss ebenfalls zu höheren Teilepreisen.

US 3 932 093 A und FR 2 244 609 A1 offenbaren weitere Verfahren und komplex aufgebaute Expander-Werkzeuge zum Herstellen einer Injektionsvorrichtung.

Der Erfindung liegt die Aufgabe zugrunde, ein Verfahren zum Herstellen einer Injektionsvorrichtung mit einem Bypasskanal zu schaffen, das einfach und zuverlässig zu handhaben ist und die Kosten pro hergestellter Injektionsvorrichtung senkt.

Diese Aufgabe wird durch ein Verfahren zum Herstellen einer Injektionsvorrichtung mit einem Bypasskanal gelöst, das die Merkmale des Anspruchs 1 umfasst.

Ferner liegt der Erfindung die Aufgabe zugrunde, ein Werkzeug zum Herstellen einer Injektionsvorrichtung mit Bypasskanal zu schaffen, mit dem auf einfache, zuverlässige und kostengünstige Weise der Bypasskanal in der Injektionsvorrichtung ausgeformt werden kann.

Diese Aufgabe wird durch ein Werkzeug gelöst, dass die Merkmale des Anspruchs 5 umfasst.

Das erfindungsgemäße Verfahren zum Herstellen einer Injektionsvorrichtung mit einem Bypasskanal zeichnet sich dadurch aus, dass zunächst ein Vorformling in ein Werkzeug eingelegt wird. Dabei liegt der Vorformling teilweise mit einer zylindrischen Außenfläche eines zylinderförmigen Abschnitts im Werkzeug auf. Anschließend erfolgt ein Erwärmen eines Teilbereichs des zylinderförmigen Abschnitts. Parallel dazu kann ein Stempel in den zylinderförmigen Abschnitt im Wesentlichen in axialer Richtung eingeschoben werden. Wenn sich der Stempel in der Position zum erwärmten Teilbereich befindet, wird der Stempel in radialer Richtung auf eine zylindrische Innenfläche des zylinderförmigen Abschnitts des Vorformlings verschoben. Der Stempel wird dann in den erwärmten Teilbereich des zylinderförmigen Abschnitts eingedrückt und dabei der Bypasskanal durch plastische Verformung des erwärmten Teilbereichs ausgeformt.

Das erfindungsgemäße Verfahren hat den Vorteil, dass der Bypasskanal mittels dem Stempel, z.B. nach dem Spritzgussprozess, geformt wird. Durch die Benutzung des Stempels können aufwendige Betriebsmittel gespart werden und im Körper der Injektionsspritze entstehen keine Partikel oder Trennungen, die zu Verunreinigungen beim fertigen Produkt führen würden.

Der Teilbereich des zylinderförmigen Abschnitts wird mit einer Wärmequelle erwärmt, bzw. erhitzt. Mit der Wärmequelle wird das Material des Teilbereichs auf eine derartige Temperatur gebracht, dass das Material des Vorformlings plastisch durch den Druck des Stempels in radialer Richtung verformt werden kann. Das Erwärmen des Teilbereichs des zylinderförmigen Abschnitts kann mittels der Wärmequelle durch Strahlungswärme und/oder Kontaktwärme durchgeführt werden.

Der Vorformling für die Injektionsvorrichtung kann dabei aus Cyclo-Olefin-Copolymer (COC), Cyclic-Olefin-Polymer (COP) oder aus Polypropylen (PP) spritzgegossen werden.

Durch das Eindrücken des Stempels in den erwärmten Teilbereich des zylinderförmigen Abschnitts erfolgt eine plastische Deformation, durch die ein Hinterschnitt im Teilbereich des zylinderförmigen Abschnitts ausgebildet wird. Der Hinterschnitt definiert den Bypasskanal, der von der zylinderförmigen Innenfläche des Vorformlings nach außen gedrückt wird.

Das Werkzeug zur Herstellung einer Injektionsvorrichtung mit Bypasskanal zeichnet sich auch dadurch aus, dass es derart ausgebildet ist, dass ein Vorformling der Injektionsvorrichtung im Werkzeug in zumindest einem zylinderförmigen Abschnitt unterstützt ist. Eine Wärmequelle im Werkzeug ist zum Erwärmen eines Teilbereichs des zylinderförmigen Abschnitts des Vorformlings gegenüber einer zylindrischen Außenfläche des zylinderförmigen Abschnitts angeordnet. Ferner umfasst das Werkzeug einen Stempel, der in den Vorformling in einer axialen Richtung einfahrbar ist. Ebenso ist der Stempel derart ausgebildet, dass er im Vorformling in einer radialen Richtung zum erwärmten Teilbereich hin verfahrbar ist.

Die Wärmequelle kann als eine Kontaktwärmequelle und/oder als eine Strahlungswärmequelle ausgebildet sein. Zur Positionierung des Vorformlings im Werkzeug, hat dieses einen Anschlag für eine Handhabe des Vorformlings ausgebildet. Dies hat den Vorteil, dass der Vorformling mit dem zu erwärmenden Teilbereich reproduzierbar gegenüber der Wärmequelle des Werkzeugs positioniert ist.

Der Stempel selbst ist an einer Handhabe angebracht, mittels der der Stempel in den Vorformling in der axialen Richtung einfahrbar ist. Ebenso ist über die Handhabe der Vorformling in der radialen Richtung zum erwärmten Teilbereich hin verfahrbar. Über die Handhabe des Stempels kann auf den erwärmten Teilbereich mit dem Stempel ein derartiger Druck ausgeübt werden, dass der Stempel in dem erwärmten Material des zylinderförmigen Bereichs, bzw. des Teilbereichs des Vorformlings den Basiskanal formt. Nach dem Erkalten kann die Injektionsvorrichtung mit dem ausgeformten Bypasskanal aus dem Werkzeug entnommen werden.

Weitere Vorteile und vorteilhafte Ausgestaltungen der Erfindung sind Gegenstand der nachfolgenden Figuren, sowie deren Beschreibungsteile.

Es zeigen im Einzelnen:
- **Figur 1**: eine Schnittansicht eines Vorformlings entlang einer Längsachse.
- **Figur 2**: eine Schnittansicht des in Figur 1 dargestellten Vorformlings, der im Werkzeug positioniert ist;
- **Figur 3**: eine Schnittansicht des Vorformlings, der im Werkzeugs positioniert ist und bei dem der Bypasskanal ausgeformt wird; und
- **Figur 4**: eine Schnittansicht der erfindungsgemäßen Injektionsvorrichtung mit dem ausgeformten Bypasskanal.

Für gleiche oder gleich wirkende Elemente der Erfindung werden identische Bezugszeichen verwendet. Das dargestellte Ausführungsbeispiel für die Ausformung eines Bypasskanals für eine Injektionsvorrichtung stellt lediglich eine mögliche Ausführungsform dar, wie das erfindungsgemäße Verfahren ausgestaltet sein kann. Dies soll nicht als eine Beschränkung der Erfindung aufgefasst werden.

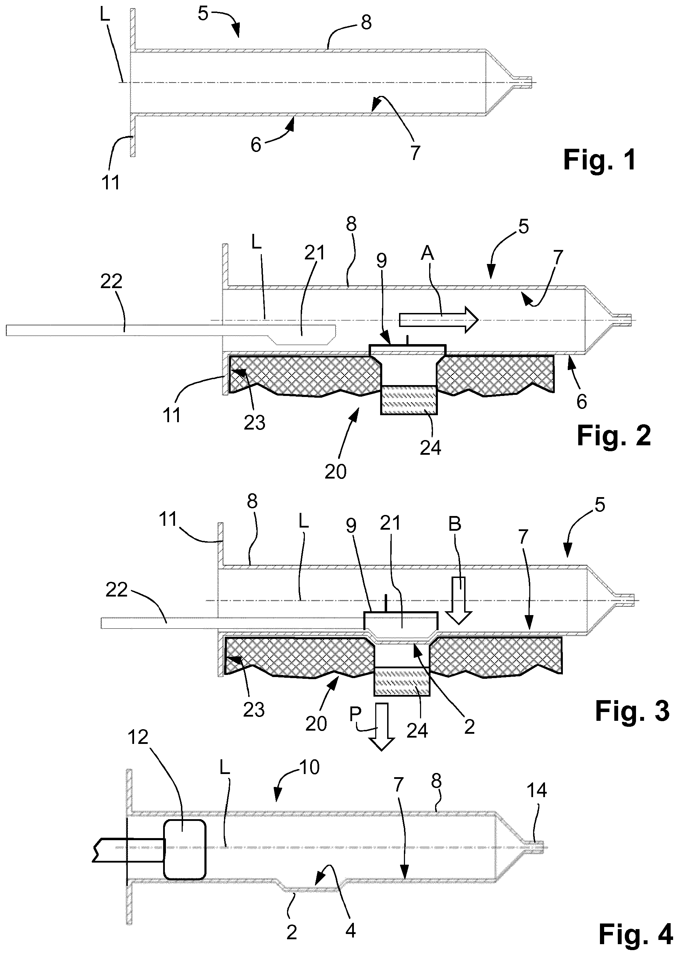

**Figur 1** zeigt eine Schnittansicht eines Vorformlings 5 für die Herstellung einer Injektionsvorrichtung 10 mit einem Bypasskanal 2 (siehe Figur 4). Der Vorformling 5 besteht aus einem zylinderförmigen Abschnitt 8, der entlang einer Längsachse L ausgerichtet ist. Der zylinderförmige Abschnitt 8 definiert eine zylindrische Außenfläche 6 und eine zylindrische Innenfläche 7. Ebenso hat der Vorformling 5 eine Handhabe 11 ausgebildet, die beim Herstellungsprozess für die Positionierung des Vorformlings 5 in einem Werkzeug 20 (siehe Figur 2) herangezogen werden kann.

**Figur 2** zeigt ebenfalls eine Schnittansicht entlang der Längsachse L des Vorformlings 5. Der Vorformling 5 ist hier bereits in einem speziell ausgebildeten Werkzeug 20 positioniert. Das Werkzeug definiert einen Anschlag 23, der mit der Handhabe 11 des Vorformlings 5 zusammenwirkt. Dadurch wird eine reproduzierbare Positionierung des Vorformlings 5 im Werkzeug 20 erreicht. Das Werkzeug 20 umfasst ebenfalls eine Wärmequelle 24, die zum Erwärmen eines Teilbereichs 9 des zylinderförmigen Abschnitts 8 des Vorformlings 5 geeignet ist. Die Wärmequelle 24 kann dabei in Form einer Kontaktwärmequelle und/oder einer Strahlungswärmequelle ausgebildet sein. Der Vorformling 5 liegt somit mit einer zylindrischen Außenfläche 6 des zylindrischen Abschnitts 8 im Werkzeug auf. Die Wärmequelle 24 erwärmt dabei den Teilbereich 9 des zylinderförmigen Abschnitts 8 auf eine Temperatur, so dass das Material des Vorformlings 5 plastisch deformiert werden kann. Gleichzeitig zur Erwärmung des Teilbereichs 9 des zylinderförmigen Abschnitts 8 kann ein Stempel 21 in axialer Richtung A in den Vorformling 5 eingeführt werden. Bevorzugt ist dabei der Stempel 21 an einer Handhabe 22 angebracht.

**Figur 3** zeigt die Situation, dass der Stempel 21 in einer radialen Richtung B auf den Teilbereich 9 des zylinderförmigen Abschnitts 8 zubewegt wurde. Bevorzugt ist der Vorformling 5 aus einem thermoplastischen Kunststoff spritzgegossen. Im Besonderen werden für den Vorformling 5 die Materialien COC, COP und PP verwendet. Die Bewegung des Stempels 21 in radialer Richtung B wird im Wesentlichen durch die Handhabe 22 bewirkt. Sobald der Stempel 21 die zylindrische Innenfläche 7 des zylinderförmigen Abschnitts 8 erreicht, wird über die Handhabe 22 auf den Stempel 21 ein Druck P in radialer Richtung B ausgeübt, der für eine plastische Verformung des erwärmten Teilbereichs 9 sorgt.

**Figur 4** zeigt eine Schnittansicht der mit dem erfindungsgemäßen Verfahren hergestellten Injektionsvorrichtung 10 entlang der Längsachse L. Der Bypasskanal 2 ist dabei im zylinderförmigen Abschnitt 8 ausgebildet. Dabei ist der zylinderförmige Abschnitt 8 von der zylindrischen Innenfläche 7 her im Teilbereich 9 (siehe Figur 2 + 3) nach außen gewölbt, so dass dadurch der zylinderförmige Abschnitt 8 der Injektionsvorrichtung 10 einen Hinterschnitt 4 ausgebildet hat. In die Injektionsvorrichtung 10 kann ferner noch ein Kolben 12 eingesetzt werden, mit dem das in der Injektionsvorrichtung 10 vorhandene Material durch eine Düse 14 der Injektionsvorrichtung 10 ausgebracht werden kann.

### Bezugszeichenliste

- 2: Bypasskanal
- 4: Hinterschnitt
- 5: Vorformling
- 6: zylindrische Außenfläche
- 7: zylindrische Innenfläche
- 8: zylinderförmiger Abschnitt
- 9: Teilbereich
- 10: Injektionsvorrichtung
- 11: Handhabe
- 12: Kolben
- 14: Düse
- 20: Werkzeug
- 21: Stempel
- 22: Handhabe
- 23: Anschlag
- 24: Wärmequelle
- A: axiale Richtung
- B: radiale Richtung
- L: Längsachse
- P: Druck

## Patentansprüche

1. Verfahren zum Herstellen einer Injektionsvorrichtung (10) mit einem Bypasskanal (2), umfassend die folgenden Schritte:
• Einlegen eines Vorformlings (5) in ein Werkzeug (20), so dass der Vorformling (5) teilweise mit einer zylindrischen Außenfläche (6) eines zylinderförmigen Abschnitts (8) im Werkzeug (20) aufliegt;
**gekennzeichnet durch**
• Erwärmen eines Teilbereichs (9) des zylinderförmigen Abschnitts (8) mit einer Wärmequelle (24) des Werkzeugs (20) auf eine Temperatur, so dass das Material des Vorformlings (5) plastisch deformiert werden kann;
• Einschieben eines Stempels (21) in den zylinderförmigen Abschnitt (8), im Wesentlichen in einer axialen Richtung (A);
• Verschieben des Stempels (21) in einer radialen Richtung (B) auf eine zylindrische Innenfläche (7) des zylinderförmigen Abschnitts (8) des Vorformlings (5);
• Eindrücken des Stempels (21) in den erwärmten Teilbereich (9) des zylinderförmigen Abschnitts (8), so dass durch einen Druck (P) des Stempels (21) in der radialen Richtung (B) der Bypasskanal (2) durch plastische Verformung des erwärmten Teilbereichs (9) ausgeformt wird.

2. Verfahren nach Anspruch 1, wobei das Erwärmen des Teilbereichs (9) des zylinderförmigen Abschnitts (8) mittels Strahlungswärme und/oder Kontaktwärme durchgeführt wird.

3. Verfahren nach einem der vorangehenden Ansprüche, wobei der Vorformling (5) aus Cyclo-Olefin-Copolymer, Cyclic-Olefin-Polymer oder Polypropylen spritzgegossen ist.

4. Verfahren nach einem der vorangehenden Ansprüche, wobei durch das Eindrücken des Stempels (21) in den erwärmten Teilbereich (9) des zylinderförmigen Abschnitts (8) durch die plastische Deformation ein Hinterschnitt (4) im Teilbereich (9) des zylinderförmigen Abschnitts (8) ausgebildet wird, wobei der Hinterschnitt (4) den Bypasskanal (2) definiert.

5. Werkzeug (20) zum Herstellen einer Injektionsvorrichtung (10) mit einem Bypasskanal (2),
**gekennzeichnet durch**
eine Auflage zum Unterstützen zumindest eines zylinderförmigen Abschnitts (8) eines Vorformlings (5),
eine Wärmequelle (24) im Werkzeug (20) zum Erwärmen eines Teilbereichs (9) des zylinderförmigen Abschnitts (8) des Vorformlings (5) gegenüber einer zylindrischen Außenfläche (6) des zylinderförmigen Abschnitts (8); und
einen Stempel (21), der in den Vorformling (5) in einer axialen Richtung (A) einfahrbar und im Vorformling (5) in einer radialen Richtung (B) zum erwärmten Teilbereich (9) hin verfahrbar ist.

6. Werkzeug (20) nach Anspruch 5, wobei die Wärmequelle (24) eine Kontakt- und/oder eine Strahlungswärmequelle ist.

7. Werkzeug (20) nach einem der vorangehenden Ansprüche 5 oder 6, wobei das Werkzeug (20) einen Anschlag (23) für eine Handhabe (11) des Vorformlings (5) ausgebildet hat, so dass der Vorformling (5) mit dem zu erwärmenden Teilbereich (9) reproduzierbar gegenüber der Wärmequelle (24) des Werkzeugs (20) positioniert ist.

8. Werkzeug (20) nach Anspruch 5, wobei der Stempel (21) an einer Handhabe (22) angebracht ist, mittels der der Stempel (21) in den Vorformling (5) in der axialen Richtung (A) einfahrbar, im Vorformling (5) in der radialen Richtung (B) zum erwärmten Teilbereich (9) hin verfahrbar und auf den erwärmten Teilbereich (9) ein Druck (P) ausübbar ist, so dass der Stempel (21) den Bypasskanal (2) formt.

## Claims

1. Method for producing an injection device (10) having a bypass channel (2), comprising the following steps:
placing a preform (5) in a tool (20) so that the preform (5) partially rests with a cylindrical outer surface (6) of a cylindrical portion (8) in the tool (20);
**characterized by**
heating a section (9) of the cylindrical portion (8) with a heat source (24) of the tool (20) to a temperature so that the material of the preform (5) can be plastically deformed;
inserting a punch (21) into the cylindrical portion (8), substantially in an axial direction (A);
displacing the punch (21) in a radial direction (B) onto a cylindrical inner surface (7) of the cylindrical portion (8) of the preform (5);
pressing the punch (21) into the heated section (9) of the cylindrical portion (8) so that, by a pressure (P) of the punch (21) in the radial direction (B), the bypass channel (2) is formed by plastic deformation of the heated section (9).

2. Method according to claim 1, wherein the heating of the section (9) of the cylindrical portion (8) is carried out by means of radiant heat and/or contact heat.

3. Method according to one of the preceding claims, wherein the preform (5) is injection-molded from cyclo-olefin copolymer, cyclic-olefin polymer or polypropylene.

4. Method according to one of the preceding claims, wherein, by pressing the punch (21) into the heated section (9) of the cylindrical portion (8), an undercut (4) is formed in the section (9) of the cylindrical portion (8) by plastic deformation, wherein the undercut (4) defines the bypass channel (2).

5. Tool (20) for producing an injection device (10) having a bypass channel (2), **characterized by**
a support for supporting at least one cylindrical portion (8) of a preform (5),
a heat source (24) in the tool (20) for heating a section (9) of the cylindrical portion (8) of the preform (5) relative to a cylindrical outer surface (6) of the cylindrical portion (8); and
a punch (21) movable into the preform (5) in an axial direction (A) and movable in the preform (5) in a radial direction (B) toward the heated section (9).

6. Tool (20) according to claim 5, wherein the heat source (24) is a contact and/or a radiant heat source.

7. Tool (20) according to one of the preceding claims 5 or 6, wherein the tool (20) has formed a stop (23) for a handle (11) of the preform (5), so that the preform (5) with the section (9) to be heated is reproducibly positioned relative to the heat source (24) of the tool (20).

8. Tool (20) according to claim 5, wherein the punch (21) is attached to a handle (22), by means of which the punch (21) can be moved into the preform (5) in the axial direction (A), can be moved in the preform (5) in the radial direction (B) toward the heated section (9), and can exert a pressure (P) on the heated section (9) so that the punch (21) forms the bypass channel (2).

## Revendications

1. Procédé de fabrication d'un dispositif d'injection (10) avec un conduit de dérivation (2), comprenant les étapes suivantes :
mise en place d'une préforme (5) dans un outil (20), de telle manière que la préforme (5) repose en partie dans l'outil (20) par une surface extérieure cylindrique (6) d'une partie cylindrique (8) ;
**caractérisé en ce qu'**il comprend
le chauffage d'une zone (9) de la partie cylindrique (8) avec une source de chaleur (24) de l'outil (20) jusqu'à une température telle que le matériau de la préforme (5) puisse subir une déformation plastique ;
l'insertion d'un poinçon (21) dans la partie cylindrique (8), sensiblement dans une direction axiale (A) ;
le déplacement du poinçon (21) dans une direction radiale (B) vers une surface intérieure cylindrique (7) de la partie cylindrique (8) de la préforme (5) ;
l'enfoncement du poinçon (21) dans la zone chauffée (9) de la partie cylindrique (8), de sorte qu'une pression (P) du poinçon (21) dans la direction radiale (B) forme le conduit de dérivation (2) par la déformation plastique de la zone chauffée (9).

2. Procédé selon la revendication 1, dans lequel le chauffage de la zone (9) de la partie cylindrique (8) est réalisé au moyen de chaleur rayonnée et/ou de chaleur de contact.

3. Procédé selon l'une des revendications précédentes, dans lequel la préforme (5) est moulée par injection en copolymère de cyclo-oléfine, en polymère d'oléfine cyclique ou en polypropylène.

4. Procédé selon l'une des revendications précédentes, dans lequel l'enfoncement du poinçon (21) dans la zone chauffée (9) de la partie cylindrique (8) forme, par la déformation plastique, une contre-dépouille (4) dans la zone (9) de la partie cylindrique (8), laquelle contre-dépouille (4) définit le conduit de dérivation (2).

5. Outil (20) pour la fabrication d'un dispositif d'injection (10) avec un conduit de dérivation (2), **caractérisé en ce qu'**il comporte
un appui pour soutenir au moins une partie cylindrique (8) d'une préforme (5),
une source de chaleur (24) dans l'outil (20) pour chauffer une zone (9) de la partie cylindrique (8) de la préforme (5) par rapport à une surface extérieure cylindrique (6) de la partie cylindrique (8) et
un poinçon (21) qui peut être introduit dans la préforme (5) dans une direction axiale (A) et déplacé dans la préforme (5) dans une direction radiale (B) vers la zone chauffée (9).

6. Outil (20) selon la revendication 5, dans lequel la source de chaleur (24) est une source de chaleur de contact et/ou rayonnée.

7. Outil (20) selon l'une des revendications 5 ou 6, dans lequel l'outil (20) comporte une butée (23) formée pour un élément de manipulation (11) de la préforme (5), de sorte que la préforme (5) avec la zone (9) à chauffer est positionnée de façon reproductible par rapport à la source de chaleur (24) de l'outil (20).

8. Outil (20) selon la revendication 5, dans lequel le poinçon (21) est disposé sur un élément de manipulation (22) au moyen duquel le poinçon (21) peut être introduit dans la préforme (5) dans la direction axiale (A), déplacé dans la préforme (5) dans la direction radiale (B) vers la zone (9) chauffée et peut exercer sur la zone (9) chauffée une pression (P) telle que le poinçon (21) forme le conduit de dérivation (2).
